Europäisches Patentamt

European Patent Office

Office européen des brevets

(19)

(11) Publication number: **0 506 961 B1**

# EUROPEAN PATENT SPECIFICATION

(12)

(45) Date of publication of patent specification: **20.12.95**

(51) Int. Cl.⁶: **A61K 7/00**, A61K 31/375, A61K 7/48

(21) Application number: **90915811.5**

(22) Date of filing: **26.10.90**

(86) International application number:
**PCT/JP90/01383**

(87) International publication number:
**WO 92/07544 (14.05.92 92/11)**

(54) **EXTERNAL PREPARATION FOR SKIN**

(73) Proprietor: **SHISEIDO COMPANY LIMITED**
**7-5-5, Ginza**
**Chuo-ku**
**Tokyo 104-10 (JP)**

Proprietor: **Senju Pharmaceutical Co., Ltd.**
**5-8, Hiranomachi 2-chome,**
**Chuo-Ku**
**Osaka-shi,**
**Osaka 541 (JP)**

(72) Inventor: **TOKUE, Wataru, 6-8-308, Nagatadai**
**Minami-ku**
**Yokohama-shi**
**Kanagawa 232 (JP)**
Inventor: **ITO, Kenzo, 2-2-26, Namiki**
**Sagamihara-shi**
**Kanagawa 229 (JP)**
Inventor: **TOMINAGA, Naoki, Hanatubakiryo**
**338, Nippacho**
**Kouhoku-ku**
**Yokohama-shi**
**Kanagawa 223 (JP)**

(43) Date of publication of application:
**07.10.92 Bulletin 92/41**

(45) Publication of the grant of the patent:
**20.12.95 Bulletin 95/51**

(84) Designated Contracting States:
**DE FR GB IT**

(56) References cited:
**EP-A- 0 127 471**      **EP-A- 0 236 120**
**JP-A-49 086 554**      **JP-A-60 078 913**
**JP-A-62 129 212**      **JP-A-62 187 470**
**US-A- 5 053 222**

**Sachio Imai, Jap. J. Pharmacol., 17, 317**
**(1967).**

**YAKUJI-KENKYUKAI "Monthly Pharmaceutical Affairs" Vol. 25, No. 9 (1983) issued by Yakuji-Jihosha, "Metabolism on Skin" (Ikuo Koyama), right column, p. 27, lines 5 to 7.**

Rank Xerox (UK) Business Services
(3.10/3.09/3.3.3)

(74) Representative: **Heim, Hans-Karl, Dipl.-Ing. et al**
**Weber & Heim**
**Patentanwälte**
**Irmgardstrasse 3**
**D-81479 München (DE)**

## Description

[Technical Field]

The present invention relates to an external preparation and more particularly, to an external preparation which is applied to the skin to resist cutaneous aging.

[Background Art]

The epidermis of the skin becomes thin with aging, and suffers from symptoms such as the reduction in the production of keratin.

One of the important factors affecting cutaneous aging is age in a broad view, but more direct causes are dryness, oxidization by active oxygen, damage by ultraviolet rays (especialy UV-A ultravioletrays which reaches corwm ) and the like.

Various methods are conventionally taken in order to resist cutaneous aging. For example, external preparations applied to the skin containing a blend of various humectants for resisting cutaneous aging due to dryness, those containing an antioxidant such as vitamin E for resisting cutaneous aging due to oxidization and those containing an absorbing agent for resisting cutaneous aging due to ultraviolet rays are conventionally used.

Such conventional cutaneous aging resisting means are no better than a symptomatic treatment, and cannot produce a sufficient cutaneous aging resisting effect.

[Disclosure of the Invention]

Accordingly, it is an object of the present invention to eliminate the above-described problems in the prior art and to provide an external preparation which is capable of resisting cutaneous aging more efficiently.

EP-A-0 127 471 dicloses cosmetic compositions comprising DL-$\alpha$-tocopherol 2-L-ascorbic phosphoric diester but no UV absorbing agents.

JP-A-62129212 describes cosmetic compositions comprising L-ascorbic acid 2-phosphate and a 2-hydroxybenzophenone derivative.

The prior art cosmetic compositions disclosed in said documents are not fully satisfactory as regards anti-aging effect and particularly the suppression of collagen crosslinking in the skin.

As a result of studies undertaken by the present inventors so as to achieve this aim, it has been found that an excellent cutaneous aging resisting effect is produced by combining a specific tocopherol derivative and an ultraviolet absorbing agent. The present invention has been achieved on the basis of the finding.

It is well known that the cross-linking of the collagen in the skin increases with aging (Cutaneous Aging, edited by Albert M. Kligman and Yoshio Takase, UNIVERSITY OF TOKYO PRESS, pp. 263 to 274, 1988 and Sugiyama T., Fujimoto D., Arai C., and Hasegawa M., Biomed. Res. 8: pp.349 to 351, 1987).

It is also known that collagen is cross-linked by ultraviolet rays (Fujimori E., FEBS Lett 235 (1 to 2) pp. 98 to 102, 1988).

Accordingly, the present inventors have aimed at production of an external preparation which resists cutaneous aging by suppressing cross-linking of the collagen in the skin.

The external preparation set forth in the Claim is characterized in that it comprises DL-$\alpha$-tocopherol 2-L-ascorbic phosphoric diester and/or a salt thereof and at least one ultraviolet absorbing agent in amounts of 0.005 to 0.2 wt% of DL-$\alpha$-tocopherol 2-L-ascorbic phosphoric diester and/or a salt thereof, and 0.01 to 15.0 wt% of ultraviolet absorbing agent.

The structure of the present invention will be explained in detail hereinunder.

The amount of DL-$\alpha$-tocopherol 2-L-ascorbic phosphoric diester (hereinunder referred to as "EPC") and/or a salt thereof in an external preparation for being applied to the skin accordingly to the present invention is 0.005 to 0.2 wt%.

Example of a preferred salt of EPC are alkali metal salts such as sodium and potassium, and alkali earth metal salts such as calcium and magnesium of EPC (hereinunder referred to as EPC-Na and the like).

If the amount of EPC is less than 0.005 wt%, it is sometimes impossible to obtain a sufficient cutaneous aging resisting effect. Addition of more than 0.2 wt% of EPC hardly increases the cutaneous aging resisting effect.

In the present invention, at least one ultraviolet absorbing agent is used in addition to EPC or a salt of EPC.

EP 0 506 961 B1

As the ultraviolet absorbing agent, ultraviolet absorbing agents which are permitted as ingredients of ordinary cosmetics are used as occasion demands. Examples thereof are:

cinamic acid ultraviolet absorbing agents such as 2-ethoxyethyl paramethoxy cinnamate, isopropyl paramethoxy cinnamate, diisopropyl cinnamate, ethylhexyl paramethoxy cinnamate, glyceryl diparamethoxy cinnamate mono-2-ethyl hexanoate and octyl methoxy cinnamate;

benzoylmethane ultraviolet absorbing agents such as butylmethoxybenzoylmethane and 4-tert-butyl-4'-methoxy-dibenzoylmethane;

bezophenone ultraviolet absorbing agents such as glyceryl-mono-2-ethylhexanoyl-di-paramethoxybenzophenone, 2-2'-dihydroxy-4-methoxybenzophenone, 2-2'-dihydroxy-4, 4'-dimethoxybenzophenone, 2-hydroxy-4-methoxybenzophenone and sodium 2-hydroxy-4-methoxybenzophenone-5-sulfonate;

benzoic acid ultraviolet absorbing agents such as methyl orthoaminobenzoate, 2-ethylhexyl-para-dimethyl aminobenzoate and octyl paradimethyl aminobenzoate;

benzoate ultraviolet absorbing agents such as grycelyl paraaminobenzoate, amyl-para-dimethyl aminobenzoate and ethyl-4-bishydroxy propyl aminobenzoate;

and

other ultraviolet absorbing agents such as 2-ethylhexyl-2-cyano-3, 3'-diphenyl acrylate, digalloyl trioleate, 2-ethylhexyl salicylate, homomethyl salicylate, guaiazulene and urocanic acid.

The amount of ultraviolet absorbing agent added is different depending upon the type of ultraviolet absorbing agent, but it is generally 0.01 to 15.0 wt% of the total amount of external preparation.

If the amount of ultraviolet absorbing agent is less than 0.01 wt%, the synergistic effect thereof and EPC is sometimes insufficient. Addition of more than 15.0 wt% of ultraviolet absorbing agent hardly increases the cutaneous aging resisting effect.

In addition to the above-described essential ingredients, it is possible to add, if necessary, other ingredients which are used for ordinary cosmetics and drugs or quasi-drugs for application to skin. Those ingredients are, for example, vitamin A's such as vitamin A oil, retinol and retinol acetate; vitamin $B_2$'s such as riboflavin, riboflavin butyrate and flavin adenine dinucleotide; vitamin $B_6$'s such as pyridoxine hydrochloride and pyridoxine dioctanoate; vitamin C's such as L-ascorbic acid, dipalmitate L-ascorbate, Na L-ascorbate-2-sulfate; pantothenic acids such as calcium pantothenate, D-pantothenyl alcohol, pantothenyl ethyl ether and acetylpantothenyl ethyl ether; vitamin D's such as ergocalciferol and cholecalciferol; nicotinic acids such as nicotinic acid, nicotinic acid amide, benzyl nicotinate; vitamin E's such as $\alpha$-tocopherol, tocopherol acetate, DL-$\alpha$-tocopherol nicotinate and DL-$\alpha$-tocopherol succinate; other vitamins such as vitamin P and biotin; amino acids and derivatives thereof such as glycine, alanine, valine, leucine, isoleucine, serine, threonine, aspartic acid and salts thereof, glutamic acid and salts thereof, lysine, arginine, cysteine, methionine, phenylalanine, tyrosine, histidine, tryptophane, proline, N-acyl acidic amino acid salts such as diethyl-N-palmitoyl L asparaginate and sodium N-coconut oil fatty acid-L-glutamate; acyl neutral amino acid salts such as coconut oil fatty acid-sarcosine triethanol amine and laurolylmethyl-$\beta$-alanine sodium; pyrrolidonecarboxylic acid and salts thereof, POE (40) hardened castor oil monopyrrogultamic monoisostearic diester and coconut oil fatty acid-L-ethyl arginate-DL-pyrrolidonecarboxylate; oil contents such as avocado oil, palm oil, peanut oil, beef tallow, rice bran oil, jojoba oil, evening primrose oil, carnauba wax, lanolin, liquid paraffin, squalane, isostearyl palmitate, isostearyl alcohol and glycerin tri-2-ethylhexanate; humectants such as glycerin, sorbitol, polyethylene glycol, 1, 3-butylene glycol, collagen, hyaluronic acid, chondroitin sulfuric acid and sodium dextran sulfate; antioxidants such as sodium erisorbate and parahydroxyanisole; surfactants such as sodium stearyl sulfate, cetyl sulfate diethanol amine, cetyl trimethyl ammonium saccharin, polyethylene glycol isostearate, glyceryl arachate, diglycerin diisostearate and phospholipid; antiseptic agents such as ethyl para-hydroxybenzoate and butyl para-hydroxybenzoate; antiphlogistic agents such as glycyrrhizic acid, gly cyrrhetic acid, salicylic acid derivative, hinokitiol, zinc oxide and allantoin; skin beautifiers such as extract of placenta, glutathione and extract of creeping saxifrage; various extracts such as extracts of phellodendron bark, goldthread, peony, Japanese green gentian, birch, sage, loquat, ginseng, aloe, mallow, iris, grape, coix seed, dishcloth gourd, lily, saffron, Cnidium officinare Makino, giner, Saint-John's wort, rosemary and garlic, vitalizers such as royal jelly, sensitizing dye, cholesterol derivatives and extract of calf's blood; blood circulation facilitators such as $\gamma$-oryzanol; anti-srborrhoeic agents such as sulfur and thianthol; thickening agents such as carboxyvinyl polymers, carboxymethyl cellulose and carboxylhydroxypropyl cellulose; perfumes; water; alcohols; coloring agents such as titanium yellow, carthamin and safflower red; and resin powder such as polyethylene and nylon powders.

The external preparation according to the present invention may take any given form. For example, it may be a soluble agent such as lotion, an emulsified agent such as milky lotion and cream, an ointment, a dispersant or an aerosol.

4

[Best Model for Carrying out the Invention]

The present invention will be explained in detail with reference to preferred embodiments. The present invention, however, is not restricted to the embodiments.

Effect of ultraviolet rays in suppressing the cross-linking of collagen

A method of obtaining the ratio of cross-linking of collagen caused by ultraviolet rays will first be explained.

Collagen was extracted from human placenta with pepsin and salted out for purification (Nishihara T., and Miyata T., Collagen Symposium 3 pp. 66-93, 1962).

The purity of collagen measured 94% by electrophoresis (Hayashi T. and Nagai Y., J. Biochem., 86 (2), pp. 453 to 459, 1979). The extracted and purified collagen (final concentration; 1 mg/ml) was held in a phosphoric acid buffer of pH 4 at 37°C to produce collagen fiber, and thereafter the collagen fiber was irradiated with ultraviolet rays (TOSHIBA FL20S.BLB lamp, peak in the UV-A region: 365 nm) at an energy of 7.0 J/cm$^2$. Each of the sample shown in Tables 1 and 2 was allowed to coexist with collagen during irradiation.

Each of the sample was produced by mixing EPC-K and 2-hydroxy-4-methoxybenzophenone as an ultraviolet absorbing agent with a phosphoric buffer as a base.

The ratio of cross-linking of the collagen irradiated with ultraviolet rays was measured by electrophoresis and a densitometer (densitometer F-808 for fluorescence, produced by COSMO corporation).

The suppression ratio of the cross-linking of collagen was obtained from the following formula:

$$\text{Suppression ratio} = 100 - \frac{\text{cross-linking degree of collagen in sample}}{\text{cross-linking degree of collagen in reference}} \times 100$$

(Refference: phosphoric acid buffer)
The results of experiments are shown in the following.

Table 1

| EPC | Ultraviolet absorbing agent | Suppression ratio |
|-----|------------------------------|-------------------|
| 0.5 | 5.0 | 80 |
| 0.3 | 5.0 | 77 |
| 0.2 | 5.0 | 82 |
| 0.1 | 5.0 | 85 |
| 0.05 | 5.0 | 84 |
| 0.01 | 5.0 | 79 |
| 0.005 | 5.0 | 72 |
| 0.001 | 5.0 | 61 |
| 0 | 5.0 | 56 |

As is obvious from Table 1, the preferable amount of EPC added is 0.005 to 0.2 wt%. When it was less than 0.005 wt%, an appropriate synergistic effect was not obtained. On the other hand, even by adding more than 0.2 wt% of EPC, the increase in the effect was not observed.

5

Table 2

| EPC | Ultraviolet absorbing agent | Suppression ratio |
|-----|-----------------------------|-------------------|
| 0.1 | 20.0 | 80 |
| 0.1 | 15.0 | 85 |
| 0.1 | 10.0 | 79 |
| 0.1 | 5.0 | 85 |
| 0.1 | 1.0 | 83 |
| 0.1 | 0.5 | 72 |
| 0.1 | 0.1 | 81 |
| 0.1 | 0.05 | 77 |
| 0.1 | 0.01 | 71 |
| 0.1 | 0.005 | 61 |
| 0 | 0 | 56 |

As is obvious from Table 2, the preferable amount of ultraviolet absorbing agent added is 0.01 to 15.0 wt%. When it was less than 0.01 wt%, an appropriate synergistic effect was not obtained. On the other hand, even by adding more than 15.0 wt% of ultraviolet absorbing agent, the increase in the effect was not observed.

As a result of the experiments, it was found that single use of either EPC or an ultraviolet absorbing agent cannot efficiently suppress the cross-linking of collagen.

A combination of another ascorbic acid or a derivative thereof did not produce the excellent effect in suppressing the cross-linking of collagen such as that described above.

Table 3

| L-ascorbic acid | Ultraviolet absorbing agent | Suppression ratio |
|-----------------|-----------------------------|-------------------|
| 0.7 | 5.0 | 56 |
| 0.5 | 5.0 | 65 |
| 0.1 | 5.0 | 63 |

Table 4

| Tocopherol | Ultraviolet absorbing agent | Suppressing ratio |
|------------|-----------------------------|-------------------|
| 0.7 | 5.0 | 40 |
| 0.5 | 5.0 | 55 |
| 0.1 | 5.0 | 58 |

Table 5

| Phosphate ascorbate | Ultraviolet absorbing agent | Suppression ratio |
|---------------------|-----------------------------|-------------------|
| 0.7 | 5.0 | 38 |
| 0.5 | 5.0 | 47 |
| 0.1 | 5.0 | 53 |

As is obvious from Tables 3 to 5, single use of either ascorbic acid or tocopherol did not affect the suppression of the cross-linking of collagen in the above-described manner. Even use of an ascorbic acid derivative such as phosphate did not produce the synergistic effect, either.

In this way, it is observed that the action of suppressing the cross-linking of collagen is furthered by the idiosyncratic synergistic action of EPC and an ultraviolet absorbing agent.

The actual cutaneous aging resisting effect was examined by using hairless mice.

9-year-week hairless mice were divided into three groups, each consisting of three mice, and they were irradiated with ultraviolet rays from TOSHIBA 32BL lamp at a dosage of $14J/cm^2$/day. 0.1ml of a sample was applied to each mouse immediately before irradiation. 10 days after irradiation, 3g of the skin was cut out and homogenized in 3% acetic acid. After incubating the homogenized skin for one night, it was subjected to centrifugal separation at a rate of 2000 rpm for 10 minutes. 5% trichloro acetic acid was added to the precipitate to effect acid hydrolysis at 90°C for 30 minutes. After centrifugal separation at 2000 rpm for 10 minutes, the supernatant liquid was dialyzed and the hydroxyproline content in the collagen was measured by a method of Stagemann and Stalder (H. Stagemann and K stalder, Clinica Chemica Acta, 19, pp. 267 to 273, 1967) to calculate the amount of cross-linking collagen.

The composition of the sample liquid was as follows.

| Basic preparation | wt% |
|---|---|
| Citric acid | 0.02 |
| Sodium Citrate | 0.08 |
| Glycerin | 5.0 |
| Ethanol | 5.0 |
| Methyl para-hydroxybenzoate | 0.1 |
| Purified water | balance |

Group A (reference)      Basic preparation
Group B (Embodiment)    Basic preparation + EPC (0.2%)
                                    + sodium 2-hydroxy 4-methoxybenzophenone 5-sulfonate (1%)
Group C (Comparison)    Basic preparation
                                    + diisostearic acid L-ascorbin (1%)
                                    + sodium 2-hydroxy 4-methoxybenzophenone 5-sulfonate

The suppression ratio of the cross-linking of collagen was calculated in the above-described method. The results are shown in Table 6.

Table 6

| | Suppression ratio |
|---|---|
| Group A | - |
| Group B | 87 |
| Group C | 50 |

Concrete examples of the composition of the external preparation of the present invention will now be shown. The amount of ingredient is shown by wt%. Each of external preparations produced the synergistic effect and the cutaneous aging resisting effect.

Example 1 Lotion

| (1) EPC | 0.05 |
| (2) Sodium 2-hydroxy 4-methoxybenzophenone 5-sulfonate | 0.1 |
| (3) Tocopherol acetate | 0.01 |
| (4) Glycerin | 4.0 |
| (5) 1,3-butylene glycol | 4.0 |
| (6) Ethanol | 8.0 |
| (7) Polyoxyethylene (60) hardened castor oil | 0.5 |
| (8) Methyl para-hydroxybenzoate | 0.2 |
| (9) Citric acid | 0.05 |
| (10) Sodium citrate | 0.1 |
| (11) Perfume | 0.05 |
| (12) Purified water | balance |

⟨Process⟩

EPC, sodium 2-hydroxy-4-methoxybenzophenone-5-sulfonate, citric acid, sodium citrate, glycerin and 1, 3-butylene glycol were dissolved in purified water. Separately from this, polyoxyethylene (60) hardened castor oil, tocopherol acetate, perfume and methyl para-hydroxybenzoate were dissolved in ethanol. The latter solution was added to the purified water solution for solubilization, and the resultant solution was filtered to obtain lotion.

Example 2 Cream

| (1) Cetostearyl alcohol | 3.5 |
| (2) Squalane | 40.0 |
| (3) Bee wax | 3.0 |
| (4) Reduced lanolin | 5.0 |
| (5) Ethyl para-hydroxybenzoate | 0.3 |
| (6) Polyoxyethylene (20) sorbitan mono palmitate | 2.0 |
| (7) Monoglyceride stearate | 2.0 |
| (8) Sodium N-stearoyl glutamate | 0.5 |
| (9) 2-hydroxy-4-methoxy-benzophenone | 0.5 |
| (10) Octyl methoxycinnamate | 1.0 |
| (11) Retinol acetate | 2.0 |
| (12) Evening primrose oil | 0.05 |
| (13) Perfume | 0.03 |
| (14) EPC-Na | 0.1 |
| (15) 1,3-butylene glycol | 5.0 |
| (16) Polyethylene glycol 1500 | 5.0 |
| (17) Purified water | balance |

⟨Process⟩

Cetostearyl alcohol, squalane, bee wax, reduced lanolin, ethyl para-hydroxybenzoate, polyoxyethylene (20) sorbitan monopalmitate, monoglyceride stearate, sodium N-stearoyl glutamate, 2-hydroxy-4-methoxy-benzophenone, octyl methoxycinnamate, retinol acetate and evening primrose oil were dissolved under heating. Separately from this, EPC-Na, 1,3-butylene glycol and polyethylene glycol 1500 were heated to 75°C. These liquids were added to purified water under stirring. After pulverizing the emulsified particles by a homomixer, the mixture was rapidly cooled under stirring to produce cream.

Example 3 Milky lotion

| (1) EPC-Mg | 0.2 |
| --- | --- |
| (2) 2-ethylhexyl para-dimethylaminobenzoate | 0.1 |
| (3) Mono-2-ethylhexyl diparamethoxycinnamate | 0.2 |
| (4) Stearic acid | 1.5 |
| (5) Cetyl alcohol | 0.5 |
| (6) Bee wax | 2.0 |
| (7) Polyoxyethylene (10) monooleate | 2.0 |
| (8) L-arginine | 0.3 |
| (9) Na L-glutamate | 0.02 |
| (10) PCA-Na | 0.05 |
| (11) Na hyaluronate | 0.01 |
| (12) Propylene glycol | 5.0 |
| (13) Glycerin | 3.0 |
| (14) Ethanol | 3.0 |
| (15) Ethyl para-hydroxybenzoate | 0.3 |
| (16) Perfume | 0.03 |
| (17) Carboxyvinyl polymer | 0.12 |
| (18) Purified water | balance |

〈Process〉

Perfume was dissolved in ethanol (alcohol phase). EPC-Mg, L-arginine, Na L-glutamate, PCA-Na, Na hyaluronate, propylene glycol, glycerin and carboxyvinyl polymer were dissolved in purified water under heating and the mixture was held at 70°C (water phase). The other ingredients were mixed and dissolved under heating, and the mixture was held at 70 °C (oil phase). The oil phase was added to the water phase for preliminary emulsification and the mixture was uniformly emulsified by a homomixer. The alcohol phase was added to the emulsion under stirring. The mixture was cooled to 30 °C under stirring to obtain milky lotion.

Example 4 Foam mask

| (1) EPC-K | 0.02 |
| --- | --- |
| (2) 4-tert-butyl-4'-methoxy-dibenzoylmethane | 0.5 |
| (3) Stearic acid | 1.0 |
| (4) Behenylic acid | 1.0 |
| (5) Self-emulsification type glycerin monostearate | 1.5 |
| (6) Polyoxyethylene monostearate | 2.5 |
| (7) Batyl alcohol | 1.5 |
| (8) Perfume | 0.05 |
| (9) Glycerin | 5.0 |
| (10) 1,3-butylene glycol | 5.0 |
| (11) Polyethylene glycol 1500 | 3.0 |
| (12) Methyl para-hydroxybenzoate | 0.1 |
| (13) Potassium hydroxide | 0.15 |
| (14) Purified water | balance |
| (15) Liquified petroleum gas | 6.0 |
| (16) Dimethyl ether | 2.0 |

⟨Process⟩

EPC-K, glycerin, 1,3-butylene glycol, polyethylene glycol 1500, methyl para-hydroxybenzoate and potassium hydroxide were added to purified water and dissolved under heating at 70°C. The other ingredients except under heating, added to the mixture and uniformly mixed. The resultant mixture was charged in a container. Finally, liquefied petroleum gas and dimethyl ether were added to the mixture as a spraying agent, thereby producing a foam mask.

Example 5 Ointment

| (1) EPC-Ca | 0.1 |
|---|---|
| (2) Octyl paradimethyl aminobenzoate | 4.0 |
| (3) Butylmethoxybenzoylmethane | 4.0 |
| (4) Tocopherol acetate | 0.5 |
| (5) Retinol palmitate | 1.0 |
| (6) Stearyl alcohol | 18.0 |
| (7) Japan wax | 20.0 |
| (8) Polyoxyethylene (10) monooleate | 0.25 |
| (9) Glycerin monostearate | 0.3 |
| (10) Vaseline | 32.0 |
| (11) Purified water | balance |

⟨Process⟩

EPC-Ca was added to purified water and the mixture was held at 70°C (water phase). The other ingredients were mixed and dissolved at 70 °C (oil phase). The oil phase was added to the water phase and the mixture was uniformly emulsified by a homomixer. The mixture was then cooled to obtain ointment.

As described above, according to the external preparation of the present invention, since DL-α-tocopherol 2-L-ascorbic phosphoric diester and/or a salt thereof and at least one ultraviolet absorbing agent are contained, it is possible to suppress the cross-linking of collagen and to produce an excellent cutaneous aging resisting effect.

**Claims**

1. An external preparation comprising 0.005 to 0.2 wt.% of DL-α-tocopherol 2-L-ascorbic phosphoric diester and/or a salt thereof, and 0.01 to 15.0 wt.% of at least one ultraviolet absorbing agent.

**Patentansprüche**

1. Zubereitung zur äußerlichen Anwendung, welche 0,005 bis 0,2 Gew.-% DL-α-Tocopherol-2-L-ascorbinsäure-phosphorsäurediester und/oder ein Salz desselben und 0,01 bis 15,0 Gew.-% mindestens eines Ultraviolett-Absorptions-mittels umfaßt.

**Revendications**

1. Préparation à usage externe comprenant de 0,005 à 0,2 % en poids de diester phosphorique de DL-α-tocophérol et d'acide 2-L-ascorbique et/ou un sel de celui-ci, et de 0,01 à 15,0 % en poids d'au moins un agent d'absorption des ultraviolets.